# EUROPEAN PATENT SPECIFICATION

(11) **EP 1 222 160 B1**
(45) Date of publication and mention of the grant of the patent: **23.07.2003**
(21) Application number: 99951030.8
(22) Date of filing: 22.10.1999
(51) Int. Cl.: C07C 209/08, C07C 211/27

(54) **PROCESS FOR PREPARING A SUBSTITUTED ALLYLAMINE DERIVATIVE AND THE SALTS THEREOF**
VERFAHREN ZUR HERSTELLUNG SUBSTITUIERTER ALLYLAMIN DERIVATIVE UND DEREN SALZE
PROCEDE DE PREPARATION D'UN DERIVE D'ALLYLAMINE SUBSTITUEE ET DE SES SELS

(43) Date of publication of application: 17.07.2002
(73) Proprietor: RICHTER GEDEON VEGYESZETI GYAR R.T., 1103 Budapest X (HU)
(72) Inventor: BOD, Péter, H-2230 Gyömrö (HU); TERDY, Lászlo, H-1192 Budapest (HU); TRISCHLER, Ferenc, DECEASED (HU); FEKECS, Eva, H-1102 Budapest (HU); DEMETER, Mária, H-1163 Budapest (HU); LAUKO, Anna, H-1196 Budapest (HU); DOMANY, György, H-7695 Budapest (HU); SZABONE KOMLOSI, Györgyi, H-1183 Budapest (HU); VARGA, Katalin, H-1093 Budapest (HU)
(74) Representative: HOFFMANN - EITLE
(86) International application number: PCT/HU99/00071
(87) International publication number: WO 01/028976

(56) References cited:
- EP-A- 0 341 048
- CH-A- 678 527
- NUSSBAUMER P ET AL: "ALLYLAMINE ANTIMYCOTICS: RECENT TRENDS IN STRUCTURE-ACTIVITY RELATIONSHIPS AND SYNTHESES" PESTICIDE SCIENCE,GB,ELSEVIER APPLIED SCIENCE PUBLISHER. BARKING, vol. 31, no. 4, 1 January 1991 (1991-01-01), pages 437-455, XP000220111 ISSN: 0031-613X

## Description

The invention relates to a new process for preparing the (E)-N-methyl-N-(1-naphtylmethyl)-6,6-dimethylhept-2-ene-4-ynyl-1-amine of formula (I) and the acid addition salts thereof.

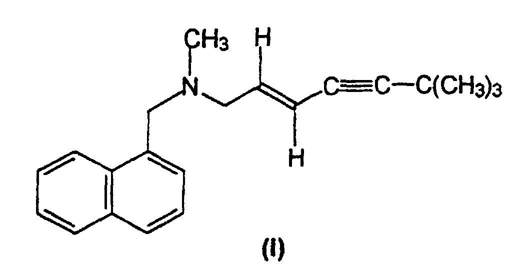

The compound of formula (I) - international nonproprietary (INN) name: terbinafine -was known first from the European Patent Specification No. 24587 (priority: 22.08.1979), as a good antifungal agent used preferably against mycosis caused by dermathophytons on the skin and on the nail. The example 16 of this patent specification describes the concrete molecule and mentions that it is a trans isomer. According to the patent specification the terbinafine was prepared also in three different chemical ways. It can be seen from the specification that the molecule was obtained always in base form - namely as the mixture of cis(Z) and trans(E) isomers. The separation may occur by column chromatography, that is not preferably used in industrial large scale production.

In a later publication (see: J. Med. Chem. **27,** 1539-1543 /1984/) the hydrochloride salt of the trans isomer was prepared from the mixture of the base by treatment of column chromatography on silica-gel and salt formation with hydrochloric acid in ethanol followed by recrystallisation. After the success of terbinafine on the market more processes were published. Thus several synthetic methods for producing the terbinafine were described in Pesticide Science, 1991, 31, pages 437-455, Elsevier Applied Science Publisher, GB. One of these methods, namely Method (b) starts 3,3-dimethyl-1-butyne. After an 1,2-addition the obtained 6,6-dimethyl-hept-1-ene-4-yn-3-ol was reacted with HBr and the obtained bromo-compound was reacted with a naphtalene-compound resulting the terbinafine. Almost the same process is described in the Swiss Patent Specification No. 678 527 or in its Hungarian equivalent Pat. No. 209 284. According to this method the hydrochloride salt of N-methyl-1-naphthalenemethylamine of formula (II) and the geometric isomeric (E:Z) mixture of 3:1 of 1-bromo-6,6-dimethyl-hept-2-ene-4-in of formula (IIIa) used as starting material.

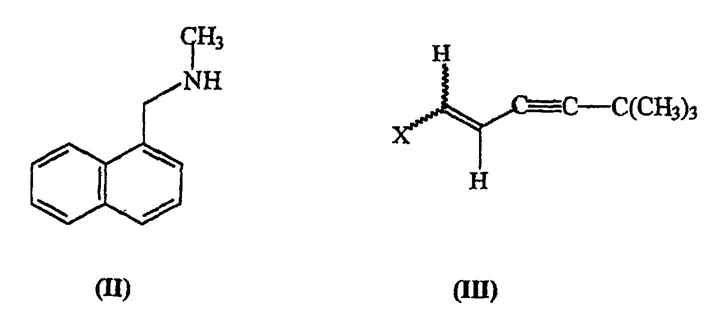

Formula (IIIa) means such formula (III) wherein X= - CH₂-Br

The essence of the procedure is that the secondary amine was alkylated by the bromo-compound of formula (IIIa), - a crude product and mixture of geometric isomers - in the presence of aqueous sodium-hydroxide. The terbinafine base was formed as the mixture of trans- and cis-isomers as an oily substance. The crude terbinafine (still a mixture of isomers) obtained by extraction with toluene and by evaporation of toluene, has the same ratio of mixture as the compound of formula (IIIa). The crude terbinafine was dissolved in ethyl acetate and hydrochloric acid gas was introduced into the solution. After a long time (4-15 hours) stirring the precipitated hydrochloride salt of transterbinafine product of formula (I) was centrifuged, washed with ethyl acetate and dried.

The disadvantages of the process are the work with the offensive and unstabile bromo-compound of formula (IIIa) and the poisoning, aromatic solvent (extraction and evaporation of toluene) and that the preparation of the hydrochloride salt of terbinafine product requires dry hydrochloric acid gas and anhydrous ethyl acetate as solvent.

The compound of formula (IIIb) - wherein in formula (III) X means a - CH₂-Cl group - is an analogue of the bromo-compound of formula (IIIa) known from the literature (see the European Patent Specification No. 341 048). It was prepared from the known 3-hydroxy-6,6-dimethyl-hept-1-ene-4-yn of formula (IV), but no characteristic data of the chloro-compound of formula (IIIb) described and as a residue of the evaporation was reacted directly with N-hydroxy-phthalimide. It is important to note that the chloro-compound of formula (IIIb) was not described elsewhere in the literature.

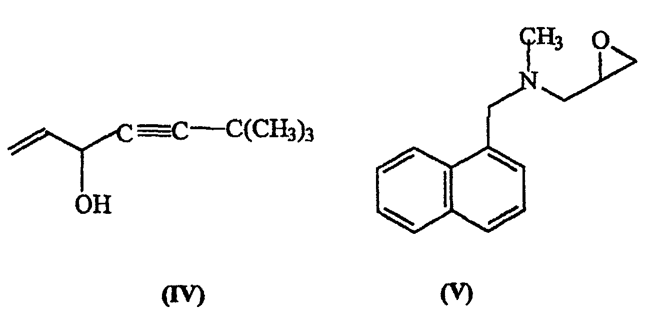

By different synthetic principle was described the preparation of terbinafine in the Canadian Patent Specification No. 2 185 599. Here the epoxide of formula (V) was obtained from the secondary amine of formula (II) with an excess of epichlorohydrin. The secondary alcohol of formula (VI) and with its dehydration a mostly undefined geometric isomeric mixture of (I) was prepared in this way.

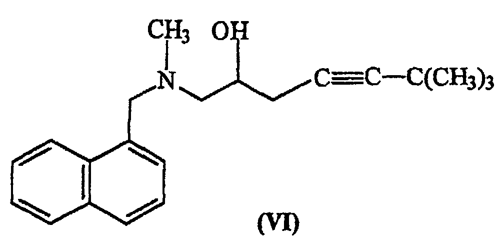

According to another version the aldehyde derivative of formula (VII) obtained also from the secondary amine of formula (II) was reacted with the phosphorus-compounds of formula (VIII) in a Wittig-type reaction and gave undefined isomeric mixture of the compound of (I) too.

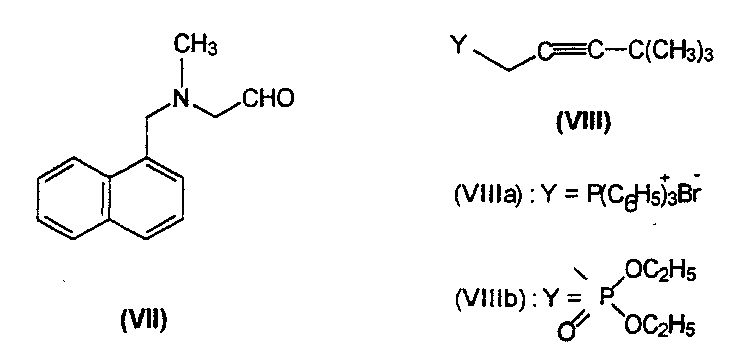

The two processes above have several disadvantages, thus:
- The requirement of a large excess of reagents (ephichlorohydrin, 3.3-dimethyl-buthyn)
- occasionally undefined amount and ratio of reagents were used
- complicated isolations by column chromatography
- the product was formed in very disadvantageous (and eventually undefined) geometric isomeric mixture (E:Z=1:9→1:1).

The aim of this invention is elaborating a process without the disadvantages of the previous known processes and applicable for large scale production.

During our experiments it was surprisingly found that without the isolation of the base form of compound of formula (I) - in a specific solvent - the acid addition salt of terbinafine may be prepared starting from the known secondary amine of formula (II) and from the secondary alcohol of formula (IV).

This observation had more unexpected results, thus:
a) reacting the secondary alcohol of formula (IV) with cc. hydrochloric acid gave the chloro-derivative of formula (IIIb) quantitatively, while by the European Patent Specification No. 341 048 this compound could be prepared by thionyl chloride with a crude yield of 88% only.
b) the quantitative formation of the compound of formula (IIIb) is surprising, because the reactivity of the aqueous hydrogen chloride used for the preparation is smaller than that of the hydrobromic acid used for the preparation of the compound of formula (IIIa).
c) However, as a result of the reaction with HCl instead of HBr, the less space requiring Cl-atom goes to the end of the molecule. The trans:cis isomeric ratio obtained is not getting worse at all, it is even a little better (3:1→3.4:1).
d) also surprising the reactivity of the choro-compound of formula (IIIb) that is similar or even a little better, than that of the bromo-analogue known from the literature. Namely the alkylation of the amine of formula (II) generated the product of formula (I) with a good yield.
e) the use of aliphatic ketones as a type of solvent gave an unexpected and surprising result. This solvent-type is excellent for the extraction of the alkylation agent of formula (IIIb) and proved to be an effective solvent by the preparation of the compound of formula (I). Furthermore using an aliphatic ketone, especially methyl isobutyl ketone as solvent, is also surprisingly advantageous, because after acidification with hydrogen chloride giving the hydrochloride salt of the end-product, both the not required hydrochloride salt of cis-isomer-basis, and the other chemical impurities of the end-product remain in the solution, so they may be removed easily.

In Table 1 are summarised the cis-isomer content of the end-products obtained in different solvents. The results demonstrate that our surprising recognition, namely the advantageous use of an aliphatic ketone, especially methyl isobutyl ketone afforded an especially low percentage of cis-isomer impurity.

Terbinafine base as a mixture of isomers of 75 w% trans- and 25 w% cis was used as starting material.

**Table 1**

| Used solvent | Cis-isomer % of the obtained terbinafine salt |
|---|---|
| toluene | 10.7 |
| n-hexane | 11.7 |
| di-isopropyl-ether | 18.9 |
| ethanol | 10.6 |
| methyl isobutyl ketone | 0.9 |

Therefore on the basis of above the aim of the invention is a new process for the preparation of the amine of formula (I) and acid addition salts thereof. This may be carried out as follows: the chloro-compound of formula (IIIb) - containing the E and Z isomers in a weight-ratio of 3.3-3.4 : 1 - was obtained reacting the secondary alcohol of formula (IV) was reacted with hydrogen chloride in a solvent. Then it was reacted with the secondary amine of formula (II) in an aliphatic ketone-type solvent in the presence of a base and optionally a iodide salt catalyst. The obtained compound of formula (I) in base form and in isomeric ratio of 3.3-3.4 : 1 was converted by aqueous hydrogen chloride directly to hydrochloride salt. The precipitated E-isomer-hydrochloride was separated and optionally the base was liberated and converted - in a known way - by another pharmaceutically acceptable acid to acid addition salt.

In a preferable version of the process according to the invention 3-7 mole of the secondary alcohol of formula (IV) was reacted with preferably 5.0-5.5 mole and preferably ice cooled cc. hydrochloric acid. Practically the reaction mixture was stirred under nitrogen overnight.

The chloro-compound of formula (IIIb) obtained as a geometric isomeric mixture - the weight-ratio of trans:cis (E:Z) is 3.3-3.4:1 - was extracted by an aliphatic ketone-type solvent, preferably methyl isobutyl ketone. Then the chloro-compound of formula (IIIb) obtained as an extract in solution was diluted by methyl isobutyl ketone and reacted with the amine of formula (II). This alkylation reaction was carried out in the presence of an amine-base at 20-80 °C, in 1-16 hours, preferably in the presence of equimolar N,N-diisopropylethylamine and 5-7 mole % iodide-salt catalyst in 3-5 hours.

The base-form of the dissolved compound of the formula (I) obtained in the phase of methyl isobutyl ketone was converted to a hydrogen-chloride salt by adding aqueous hydrochloric acid. The pH of the mixed two-phase system was adjusted to 1.0-3.0, preferably to 1.5-2.0. Then it was cooled with stirring, the precipitated solid was filtered off, washed with water and with methyl isobutyl ketone and dried.

The obtained hydrochloride salt - containing the desired E isomer - was treated with base e.g. ammonium-hydroxide in mild conditions. The base of formula (I) obtained in this way was converted by a pharmaceutically acceptable acid to acid addition salt.

One of the starting materials of the process according to our invention, the secondary alcohol of the formula (IV) is a known compound. This compound may be prepared e.g. by the EP 24 587 Patent Specification from 3,3-dimethyl-1-butyne and acrolein. (See J. Med. Chem. **27,** 1539-42 (1984))

Other starting material of the process according to our invention - the secondary amine of formula (II) - was described as a hydrochloride salt in Belstein 12 , II, 740, III. 3097, and IV. 3192.

Summarising the advantages of the process according to our invention:
1) The secondary alcohol of formula (IV) may be converted in a simple way by cc. aqueous hydrogen chloride to the alkylating chloro-compound of formula (IIIb)
2) The compound of formula (IIIb) obtained quantitatively from the reaction mixture by a single extraction with aliphatic ketone may be reacted further in the same medium. The hydrochloride of the end-product of formula (I) precipitates simply from the aqueous-ketone, two-phase reaction mixture by the final acidification by hydrochloric acid
3) The ketone, preferably methyl isobutyl ketone used by us in the reaction steps has three functions in the process: it is an extracting agent, solvent and finally cosolvent, keeping the apolar, hardly water soluble impurities of the end-product in solution.
4) Our process compared to the processes of the prior art is the most simple in large scale operations. The secondary alcohol of formula (IV) may be added to the apparatus and the hydrochloride of the compound of formula (I) - as the pure (E) trans isomer - is obtained at the end of the process.
5) While in the processes of the prior art for converting the compound of formula (I) hydrochloric acid gas was used, our process uses the more suitable aqueous hydrochloric acid.
6) In our process the hydrochloride salt of the end-product precipitates from a heterogeneous, two-phase, uper ketone, lower acid-aqueous system. The high purity of the product obtained by the process according to the invention is due to this. The total impurity of the product (see the Example 1, where it is 0.19%) less, than the value in the processes of prior art (0.3%). Moreover the end-product does not contain - because it can not - bromide impurity.
7) Our process does not require expensive, anhydrous solvents.

### Example 1

### Step A

### Preparation of 1-chloro-6,6-dimethyl-hept-2-ene-4-yn

To 13.82 g (0,1 mole) 6,6-dimethyl-hept-1-ene-4-yn-3-ol of formula (IV) 54 g (0,54 mole, 46 ml) cc. hydrochloric acid was added dropwise with stirring and ice cooling at 2-6°C. After 8 hours stirring under ice-cooling the reaction mixture was further stirred overnight (15-16 hours).

40 g (50 ml) methyl isobutyl ketone was given to the two phase system.. After separating the lower acid-water phase, the upper organic phase was extracted with 10 g water three times. Separating the aqueous phase the 1-chloro-6,6-dimethyl-hept-2-ene-4-in of formula (IIIa) was obtained practically quantitatively - in the form of the mixture of trans-cis isomers - as an acid free methyl isobutyl ketone extract.

### Step B

### (E)-N-methyl-N-(1-naphtylmethyl)-6,6-dimethylhept-2-ene-4-ynyl-1-amine-hydrochloride

To the chloro-compound of formula (IIIb) obtained as a solution of methyl isobutyl ketone in the step A, 16 g (20 ml) methyl isobutyl ketone, 17.4 g (0,1 mole) N-methyl-1-naphthalenemethylamine of formula (II), 13.0 g (0,1 mole) N,N-diisopropylethylamine, 26 g ion free water and 2.22 g (0,006 mole) tetrabutyl-ammonium-iodide was added in the given order and stirred for 4 hours at 70-80 °C. The mixture was cooled to room temperature, after diluting with methyl isobutyl ketone the lower aqueous phase was separated and to the upper organic phase 15,5 g (13 ml) cc. hydrochloric acid was added dropwise with stirring at 16-20 °C.

After the crystallisation was started, the reaction mixture was stirred for 1-2 hours at room temperature, then for further 1-2 hours at 2-6 °C. The product was filtered off, washed twice with water, once with methyl isobutyl ketone. After drying 17.5 g (53,37 %) terbinafine-hydrochloride salt was obtained (calculated for the secondary alcohol of formula (IV) used as starting material in Step A.)
Melting point: 205-207 °C
Total impurity (by HPLC method): 0.19 %

### Example 2

It was made in the same manner as the Example 1 with the difference that the reaction described in Step B is carried out without tetrabutylammonium iodide.
Yield: 16.1 g (49.1 %)
Melting point: 205-207 °C
Total impurity (by HPLC-method): 0.195%

### Example 3

The same as Example 1 with the difference that 17.5 g terbinafine-hydrochloride obtained in Step B was converted to base by aqueous ammonia. The obtained product was reacted by 5 g lactic acid and afforded 18.5 g terbinafine-lactate in this way.

### Example 4

In a 250 ml flask 14.3 g 6,6-dimethyl-1-heptene-4-yn-3-ol (96%, that is equivalent with 0.1 mole 100%) was placed. Then 54 g (46 ml, 0.54 mole) cc. hydrochloric acid was added dropwise with stirring, under nitrogen atmosphere at 0-(-2) °C. The reaction mixture was stirred for 22 hours at this temperature. Then the reaction mixture was allowed to warm to 20-25 °C and 40 ml methyl isobutyl ketone was added and stirred for 15 minutes at this temperarure. The organic layer was separated, then washed with 4x20 ml water. Then 20 ml methyl isobuthyl ketone, 17.4 g (0.1 mole) N-methyl-1-naphthylmethyl-amine, 12.9 g, (17.5 ml, 0.1 mole) N,N-diisopropylethylamine, 26 ml water and 2.2 g tetrabuthylammonium iodide was added under nitrogen over 1 hour and stirred without cooling and heating. Then it was heated to 70-80 °C and stirred for 1 hour. The reaction mixture was cooled to 20-25 °C and 40 ml methyl isobuthyl ketone was added. The organic layer was separated and 10 ml hydrochloric acid was added dropwise with stirring at 15-20 °C. It was stirred for 2 hours at this temperature after the crystallisation started. Then it was cooled to 5-10 °C and stirred for 1 hour.

The obtained crystals were filtered off, washed with 2x20 ml 5°C water, then 2x20 ml 5°C methyl isobutyl ketone. The wet product was suspended in the mixture of 100 ml methyl isobutyl ketone and 7 ml of water. 6.9 ml 25% ammonium-hydroxide was added dropwise at 20-25 °C and stirred for 15 minutes. The solution was filtered on glass filter and the layers were separated. 9.2 ml 6 N hydrochloric acid was added dropwise to the solution and stirred for 1 hour at this temperature, then further 1 hour at 5-10 °C. The product was filtered off, washed with 2x20 ml 5 °C water and 2x20 ml 5 °C methyl isobuthyl ketone.

The product was dried on the air at 40 °C.
Yield: 16.9 g (51.5 %)
Melting point: 204-205 °C
Impurity (cis-isomer): less then 0.1%

### Example 5

In a 250 ml flask 14.3 g 6.6-dimethyl-1-heptene-4-yn-3-ol was placed and then 54 g (46 ml, 0,54 mole) cc. hydrochloric acid was added dropwise with stirring, under nitrogen at 0-(-2)°C. It was stirred for 22 hours at this temperature. Then the reaction mixture was allowed to warm to 20-25 °C and 50 ml methylene chloride was added to the mixture. It was stirred for 10 minutes then the layers were separated. The organic layer was washed with 4x20 ml water and evaporated at 30 °C. The 17 g oily residue was dissolved in 50 ml acetone then 17.4 g (0.1 mole) N-methyl-1-naphthalenemethylamine, 26 ml water, 12.9 g (17.5 ml, 0.1 mole) N,N-diisopropylethylamine and 2.22 g tetrabutylammonium-iodide was added . The mixture was stirred at room temperature for 1 hour under nitrogen and further 6 hours with reflux. The obtained solution was cooled to 20-25 °C and the layers were separated. 40 ml acetone was added to the upper layer and then 10 ml cc. hydrochloric acid was added dropwise at 16-20 °C. It was stirred at this temperature for 1 hour and further 1 hour at 5-10 °C. The product was filtered, washed with 2x15 ml 5 °C water and 2x15 ml 5 °C acetone. The obtained product was dried on air at 40 °C.
Yield: 13.1 g (40%)
Melting point: 204-205 °C
Impurity (cis-isomer): 0.12%

### Example 6

In a 250 ml flask 14.3 g 6,6-dimethyl-1-heptene-4-yn-3-ol was placed and 54 g; (46 ml, 0.54 mole) cc. hydrochloric acid was added dropwise with stirring, under nitrogen, at 0-(-2) °C. It was stirred for 22 hours at this temperature. The temperature was allowed to warm to 20-25 °C and 50 ml methylene chloride was added to the mixture. It was stirred for 10 minutes and the layers were separated. The organic layer was washed with 4x20 ml water and was evaporated at 30 °C.

The oily residue was dissolved in 50 ml methyl ethyl ketone and 17.4 g (0,1 mole) N-methyl-1-naphthalenemethylamine, 2.22 g tetrabutylammonium-iodide and 26 ml water was added and the suspension was stirred for 1 hour at room temperature. It was stirred for further 2 hours with reflux, cooled to 20-25 °C and 40 ml methyl ethyl ketone was added. The organic layer was separated then at the same temperature 10 ml cc. hydrochloric acid was added and the mixture was stirred for 1 hour. The obtained suspension was stirred for 4 hours at 0-4 °C then the product was filtered off and washed with 5 ml 5 °C methyl ethyl ketone.

The obtained product was dried on air, at 40 °C.
Yield: 11.7 g (3 8%)
Melting point: 204-204 °C
Impurity (cis-isomer): 0.17%

## Claims

1. Process for the preparation of (E)-N-methyl-N-(1-naphthylmethyl)-6,6-dimethylhept-2-ene-4-ynyl-1-amine of formula (I) 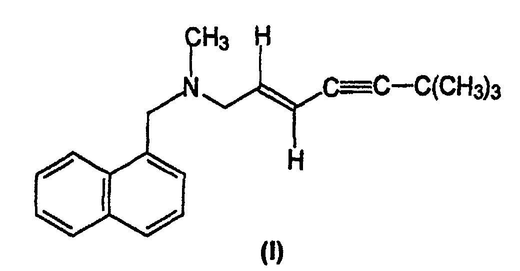 and the acid addition salts thereof wherein the secondary alcohol of formula (IV) 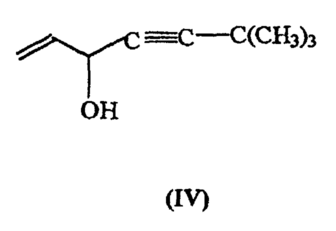 is reacted with hydrochloric acid, then the obtained the compound of formula (IIIb) 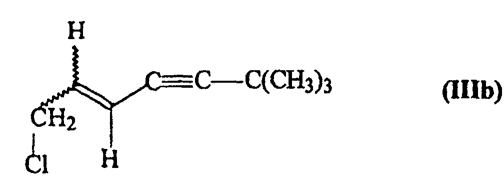
- containing the E and Z isomers of ratio 3.3-3.4, : 1 - is reacted with the secondary amine of formula (II) 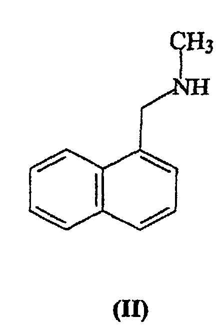 in an aliphatic ketone-type solvent in the presence of a base and optionally iodide salt catalyst, then the obtained compound of formula (I) in base form and in ratio of isomers 3.3-3.4 : 1 is converted by aqueous hydrochloric acid directly to the hydrochloride salt, the precipitated hydrochloride of the E-isomer is separated, optionally the base is liberated and converted - by a known method - to pharmaceutically acceptable acid addition salt.

2. A process according to claim 1 wherein for preparing the compound (IIIb) 3-7 mole, preferably 5-5,5 mole cc. aqueous hydrochloric acid is used.

3. A process according to claim 1 wherein the compound (IIIb) is reacted with the secondary amine of formula (II) in the presence of a base, preferably N,N-diisopropylethylamine at 20-80 °C for 0-16 hours, preferably at 70-80 °C for 3-5 hours.

4. A process according to claims 1 and 3 wherein by the reaction of compounds of formulas (II) and (IIIb) methyl isobutyl ketone is used as an aliphatic ketone-type solvent.

5. A process according to claims 1, 3 and 4 wherein the reaction of compounds of (II) and (IIIb) in methyl isobutyl ketone is carried out in the presence of 1-10 mole % iodide salt, preferably 5-7 mole % tetrabutylammonium iodide.

6. A process according to claims 1, 3, 4.and 5 wherein the compound of formula (I) obtained in methyl isobutyl ketone solution is converted to hydrochloride salt by 5-37 % hydrochloric acid to pH 1-3 at 10-30 °C, preferably by 20-37 % hydrochloric acid to pH 1.5-2.0

## Patentansprüche

1. Verfahren für die Herstellung von (E)-N-Methyl-N-(1-naphthylmethyl)-6,6-dimethylhept-2-en-4-ynyl-1-amin der Formel (I) 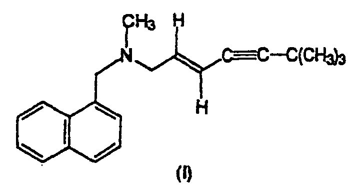 und der Säureadditionssalze davon, wobei der sekundäre Alkohol der Formel (IV) 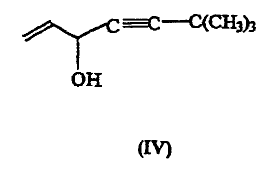 mit Salzsäure umgesetzt wird, dann die erhaltene Verbindung der Formel (IIIb) 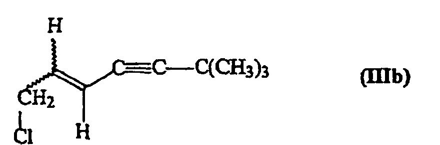
- enthaltend die E- und Z-Isomere in einem Verhältnis von 3,3-3,4:1 - mit dem sekundären Amin der Formel (II) 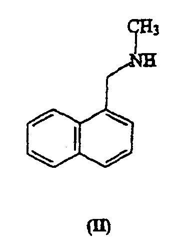 in einem aliphatischen Lösungsmittel vom Ketontyp in Gegenwart einer Base und optional einem Iodsalzkatalysator umgesetzt wird, dann die erhaltene Verbindung der Formel (I) in Basenform und in einem Isomerenverhältnis von 3,3-3,4:1 durch wässrige Salzsäure direkt in das Hydrochloridsalz umgewandelt wird, das ausgefällte Hydrochlorid des E-Isomers abgetrennt wird, optional die Base freigesetzt und - durch ein bekanntes Verfahren - in ein pharmazeutisch annehmbares Säureadditons-salz umgewandelt wird.

2. Verfahren gemäß Anspruch 1, wobei 3-7 mol, bevorzugt 5-5,5 mol, konzentrierte wässrige Salzsäure zum Herstellen der Verbindung (IIIb) verwendet werden.

3. Verfahren gemäß Anspruch 1, wobei die Verbindung (IIIb) mit dem sekundären Amin der Formel (II) in Gegenwart einer Base, bevorzugt N,N-Diisopropylethylamin, bei 20-80°C für 0-16 h, bevorzugt bei 70-80°C für 3-5 h, umgesetzt wird.

4. Verfahren gemäß Anspruch 1 und 3, wobei bei der Reaktion der Verbindungen der Formeln (II) und (IIIb) Methylisobutylketon als ein aliphatisches Lösungsmittel vom Ketontyp verwendet wird.

5. Verfahren gemäß Ansprüchen 1, 3 und 4, wobei die Reaktion der Verbindungen der Formeln (II) und (IIIb) in Methylisobutylketon in der Gegenwart von 1-10 mol% Iodsalz, bevorzugt 5-7 mol% Tetrabutylammoniumiodid, durchgeführt wird.

6. Verfahren gemäß Ansprüchen 1, 3, 4 und 5, wobei die in Methylisobutylketonlösung erhaltene Verbindung der Formel (I) durch 5-37%ige Salzsäure bei pH 1-3 bei 10-30°C, bevorzugt durch 20-37%ige Salzsäure bei pH 1,5-2,0, in das Hydrochloridsalz umgewandelt wird.

## Revendications

1. Procédé de préparation de la (E)-N-méthyl-N-(1-naphtylméthyl)-6,6-diméthylhept-2-ène-4-ynyl-1-amine de formule (I) 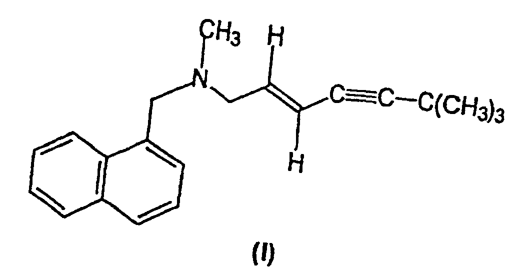 et de ses sels d'addition acide, dans lequel l'alcool secondaire de formule (IV) 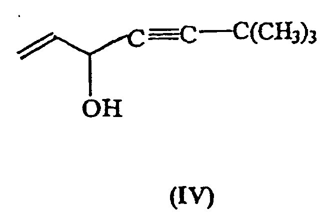 est mis en réaction avec l'acide chlorhydrique, puis le composé de formule (IIIb) obtenu 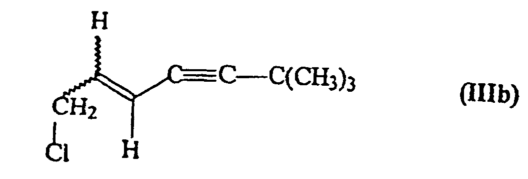 (contenant les isomères E et Z dans un rapport 3,3-3,4:1) est mis en réaction avec l'amine secondaire de formule (II) 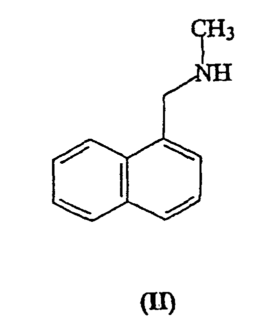 dans un solvant de type cétone aliphatique en présence d'une base et éventuellement d'un catalyseur de type sel de iodure, puis le composé de formule (I) obtenu sous forme de base et dans un rapport d'isomérie 3,3-3,4:1 est converti par l'acide chlorhydrique aqueux directement en chlorhydrate, le chlorhydrate précipité de l'isomère E est séparé, la base est éventuellement libérée et convertie (par un procédé connu) en un sel d'addition acide pharmaceutiquement acceptable.

2. Procédé selon la revendication 1, dans lequel, pour préparer le composé (IIIb), il est utilisé 3 à 7 moles, de préférence 5 à 5,5 moles d'acide chlorhydrique concentré aqueux.

3. Procédé selon la revendication 1, dans lequel le composé (IIIb) est mis en réaction avec l'amine secondaire de formule (II) en présence d'une base, de préférence la N,N-diisopropyléthylamine à 20-80°C pendant 0-16 heures, de préférence à 70-80°C pendant 3-5 heures.

4. Procédé selon les revendications 1 et 3, dans lequel, pour la réaction des composés de formules (II) et (IIIb), il est utilisé 1a méthylisobutylcétone en tant que solvant de type cétone aliphatique.

5. Procédé selon les revendications 1, 3 et 4, dans lequel la réaction des composés (II) et (IIIb) dans la méthylisobutylcétone est effectuée en présence de 1-10 moles pour cent de sel de iodure, de préférence 5-7 moles pour cent d'iodure de tétrabutylammonium.

6. Procédé selon les revendications 1, 3, 4 et 5, dans lequel le composé de formule (I) obtenu dans la solution de méthylisobutylcétone est convertie en chlorhydrate par de l'acide chlorhydrique à 5-37 % à pH 1-3 à 10-30°C, de préférence par de l'acide chlorhydrique à 20-37 % à pH 1,5-2,0.
